(19) Europäisches Patentamt
European Patent Office
Office européen des brevets

(11) Veröffentlichungsnummer: **0 129 152**
**B1**

(12) **EUROPÄISCHE PATENTSCHRIFT**

(45) Veröffentlichungstag der Patentschrift: **17.10.90**

(51) Int. Cl.⁵: **C 07 D 249/08,** A 01 N 43/64

(21) Anmeldenummer: **84106479.3**

(22) Anmeldetag: **06.06.84**

# (54) Verfahren zur Herstellung von Triazolylalkoholen.

(30) Priorität: **10.06.83 DE 3321023**

(43) Veröffentlichungstag der Anmeldung:
**27.12.84 Patentblatt 84/52**

(45) Bekanntmachung des Hinweises auf die Patenterteilung:
**17.10.90 Patentblatt 90/42**

(84) Benannte Vertragsstaaten:
**AT BE CH DE FR GB IT LI NL SE**

(56) Entgegenhaltungen:
**EP-A-0 040 350**
**EP-A-0 069 290**

(73) Patentinhaber: **BASF Aktiengesellschaft**
**Carl-Bosch-Strasse 38**
**D-6700 Ludwigshafen (DE)**

(72) Erfinder: **Sauter, Hubert, Dr.**
**Neckarpromenade**
**D-6800 Mannheim 1 (DE)**
Erfinder: **Schuster, Ludwig, Dr.**
**Weinheimer Strasse 44**
**D-6703 Limburgerhof (DE)**
Erfinder: **Pommer, Ernst-Heinrich, Dr.**
**Berliner Platz 7**
**D-6703 Limburgerhof (DE)**
Erfinder: **Ammermann, Eberhard, Dr.**
**Sachsenstrasse 3**
**D-6700 Ludwigshafen (DE)**

**EP 0 129 152 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Verfahren zur Herstellung von neuen, diastereomeren Formen der Konfiguration R*, S* von Triazolylalkoholen.

Es ist bekannt, daß Diastereomerengemische von einigen Triazolylalkoholen der Formel I fungizide Wirksamkeit besitzen (vgl. EP—A—00 40 350). Es hat sich herausgestellt, daß diese Diastereomerengemische jeweils weit überwiegend ein Diastereomer der Konfiguration R*, R* enthalten.

Es wurde nun gefunden, daß man Mischungen, enthaltend Triazolylalkohole der Formel I

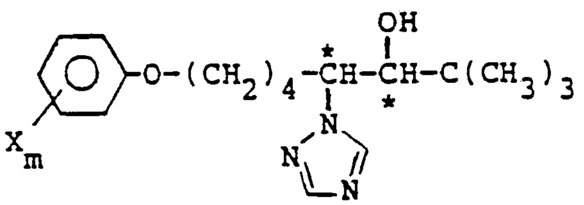

(I)

in der

X ein Wasserstoffatom, ein Halogenatom, Trifluormethyl, $C_1$- bis $C_4$-Alkyl, $C_1$- bis $C_4$-Alkoxy, gegebenenfalls substituiertes Phenyl oder gegebenenfalls substituiertes Phenoxy und

m eine ganze Zahl von 1 bis 5 bedeutet,

wobei X gleich oder verschieden sein kann, wenn m größer als 1 ist, die weniger als 30%, bezogen auf das Gesamtgemisch, an Diastereomeren, die an den beiden Chiralitätszentren die Konfiguration R*, R* haben, oder an Enantiomeren, die an den beiden Chiralitätszentren die Konfigurationen R,R oder S,S haben, enthalten, dadurch erhält, daß man Ketone der Formel II

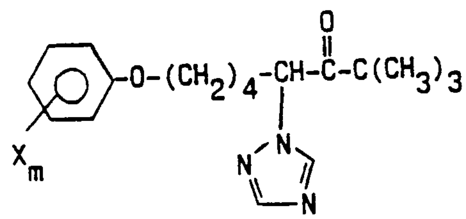

(II)

in der X und m die oben angegebene Bedeutung haben, a) mit sekundären Alkoholaten, b) mit Alkylmagnesiumhalogeniden, die einen Alkylrest mit 2 bis 6 C-Atomen und ein betaständiges Wasserstoffatom im Alkylrest enthalten, oder c) mit Wasserstoff in Gegenwart von Ruthenium oder Rutheniumderivaten und in Anwesenheit eines Verdünnungsmittels stereoselektiv reduziert.

Zur Erläuterung sei vermerkt, daß Verbindungen mit zwei verschiedenen chiralen Kohlenstoffatomen vier Stereoisomere bilden, deren Konfiguration nach dem Cahn-Ingold-Prelog-System bezeichnet werden kann (siehe z.B. D. Seebach und V. Prelog, Angew. Chem. 94, 696 (1982) und dort angegebene Literatur). Hierbei stellen zwei Enantiomere der Konfigurationen R, R und S, S ein Diastereomer mit der Bezeichnung R*, R* dar. Das andere Diastereomer mit der Bezeichnung R*, S* setzt sich aus den beiden Enantiomeren mit den Konfigurationen R, S und S, R zusammen.

In der Formel I kann der Phenoxyrest beispielsweise folgendermaßen mit $X_m$ substituiert sein:

Wasserstoffatome, 2-Fluor, 4-Fluor-, 2-Chlor-, 3-Chlor-, 4-Chlor-, 4-Brom-, 2,4-Dichlor-, 2,4,6-Trichlor-, 3,5-Dichlor-, 3-Trifluormethyl-, 4-Trifluormethyl-, 2-Propyl-(1), 3-(4-Chlorphenyl)-, 3-(2-Fluorphenoxy)-, 3-(3-Chlorphenoxy)-, 4-(3-Chlorphenyl)-, 3-tert.-Butoxy-.

Rutheniumderivate sind insbesondere Rutheniumoxidhydrat ($RuO(OH)_x$).

Reine R*,S*-Diastereomere können aus der Mischung durch einfaches Waschen der Rohprodukte mit geeigneten Lösungsmitteln, wie z.B. Diisopropylether, oder durch Umkristallisieren oder durch andere, übliche Reinigungsschritte, z.B. Chromatographie erhalten werden.

Im Gegensatz hierzu bestehen die in der EP—A—00 40 350 im einzelnen beschriebenen Beispiele für Verbindungen der oben angegebenen Formel I aus Diastereomerengemischen R*,R* und R*,S*, in denen aufgrund der Herstellung — überwiegend aus Ketonen der Formel II mit Hilfe von Natriumborhydrid — der Anteil der R*,R*-Diastereomeren weit überwiegt.

Zur stereoselektiven Reduktion der genannten Ketone der Formel II zu den erfindungsgemäßen R*,S*-Diastereomeren der Formel I in der Ausführungsform des oben erwähnten Verfahrens a) werden diese Ketone (1 Moläquivalent) mit sekundären Alkoholaten (vorzugsweise 0,3 bis 1,5 Moläquivalente), vorzugsweise des Aluminiums, wie z.B. Aluminiumisopropylat, Aluminiumbutylat-(2) oder Aluminiumcyclohexylat in Gegenwart eines Verdünnungsmittels bei 60—160°C, vorzugsweise 0,3 bis der Siedetemperatur des Verdünnungsmittels umgesetzt. Als Verdünnungsmittel eignen sich inerte organische Lösungsmittel, insbesondere Alkohole wie Isopropanol oder Cyclohexanol. Die entstandenen diastereomeren Alkoholate werden sodann mit Hilfe von Säuren in üblicher Weise zu den freien Alkoholen der Formel I hydrolysiert.

Für die stereoselektive Reduktion in der Ausführungform b) des oben erwähnten Verfahrens werden die Ketone der Formel II (1 Moläquivalent) mit Alkylmagnesiumhalogeniden, die einen Alkylrest mit 2 bis 6 C-Atomen und ein betaständiges Wasserstoffatom im Alkylrest enthalten, vorzugsweise mit 0,7—1,5 Moläquivalent, in Gegenwart eines Verdünnungsmittels bei Temperaturen zwischen 0 und 120°C, umgesetzt. Als Alkylmagnesiumhalogenide kommen z.B. Ethylmagnesiumchlorid, Isopropylmagnesiumbromid, n-Propylmagnesiumbromid oder Isobutylmagnesiumchlorid in Frage. Als Lösungsmittel für die Grignard-Reduktion kommen bevorzugt Ether in Frage, wie Diethylether, Di-n-propylether, Tetrahydrofuran oder Anisol, ferner tertiäre Amine wie N,N-Diethylanilin sowie Phosphorsäure-tris(dimethylamid). Gegebenenfalls kann man die Reaktion auch im Gemisch dieser Lösungsmittel mit aliphatischen oder aromatischen Kohlenwasserstoffen wie n-Hexan oder Toluol durchführen. Die Reaktionstemperaturen lassen sich je nach Lösungsmittel zwischen 0 und 120°C variieren, bevorzugt sind Temperaturen zwischen 30 und 100°C. Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Für die stereoselektive Reduktion in der Ausführungsform c) erfolgt die katalytische Hydrierung am besten mit Ruthenium oder Rutheniumderivaten. Man kann dies Metall auf inerte Träger niederschlagen, wobei ein Metallgehalt von 0,5 bis 10% im allgemeinen ausreichend ist. Unter Umständen kann man auch das reine Metall, zweckmäßig in fein verteilter Form einsetzen. Als besonders geeignet hat sich ein kolloid verteiltes Rutheniumoxidhydrat erwiesen, das durch Fällung aus einer wäßrigen Rutheniumtrichloridlösung bei pH = 8 gewonnen wurde. Die oxidische Form geht unter den Reaktionsbedingungen in den aktiven Katalysator über.

Als Lösungsmittel dienen vorzugsweise Ether, wie Dioxan, Tetrahydrofuran oder Glykoldimethylether.

Die Temperatur läßt sich in weiten Grenzen verändern. Als besonders geeignet erwiesen sich Temperaturen zwischen 100 und 150°C. Bei diesen Temperaturen muß man unter Druck arbeiten; es genügen Drücke von 10 bis 30 bar. Unter höheren Drücken leidet die Selektivität.

Die Kontaktmenge ist nicht kritisch und hängt neben der Aktivität von der Reaktionstemperatur und dem Wasserstoffpartialdruck ab.

Falls gewünscht, können die Gemische der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist, sofern es mit dem Substrat bzw. mit den Pflanzen verträglich ist.

Ferner lassen sich die Gemische der Formel I nach bekannten Methoden in Metallkomplexe überführen. Das kann durch Umsetzung dieser Gemische mit geeigneten Metallsalzen wie beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die Herstellung der Gemische der Formel I wird durch folgende Beispiele erläutert:

### Beispiel 1

(3-R*,4-S*)-2,2-Dimethyl-4-(1,2,4-triazol-1-yl)-8-phenoxy-octanol-(3) (Verbindung Nr. 1)

a) Zur Lösung von 7,4 g n-Propylmagnesiumbromid in 30 ml Diethylether tropft man bei Rückflußtemperatur unter Rühren eine Lösung von 12,6 g 2,2-Dimethyl-4-(1,2,4-triazol-1-yl)-8-phenoxy-octanon-(3) (siehe DE—OS 30 19 049) in 50 ml Diethylether und erhitzt die Mischung nach Beendigung der Zugabe noch 3 Stunden auf Rückflußtemperatur. Anschließend wird die Mischung auf 0°C abgekühlt und tropfenweise zunächst mit 5 ml Wasser, sodann mit 200 ml 10prozentiger, wäßriger Ammoniumchloridlösung versetzt. Die organische Phase wird abgetrennt, über Magnesiumsulfat getrocknet und im Vakuum eingeengt. Aus dem Rückstand kristallisieren nach kurzem Aufkochen mit 20 ml Diisopropylether 4,0 g der weitgehend reinen Verbindung Nr. 1 in Form farbloser Kristalle vom Fp. 132—133°C.

Die Mutterlauge enthält nach NMR-Analyse hauptsächlich nichtgesetztes Keton, wenig Verbindung Nr. 1 und sehr wenig der zur Verbindung 1 diastereomeren Form mit R*,R*-Konfiguration (Fp. 86—88°C).

b) Eine Mischung von 19 g 2,2-Dimethyl-4-(1,2,4-triazol-1-yl)-8-phenoxyoctanon-(3), 7,4 g Aluminiumtriisopropylat und 115 ml 2-Butanol wird in einer Destillationsapparatur so hoch erhitzt (ca. 100°C), daß langsam (ca. 5 ml/Stunde) ein Destillat übergeht. Von Zeit zu Zeit wird — der abdestillierenden Menge entsprechend — die siedende Mischung mit weiterem 2-Butanol aufgefüllt. Nach 70 Stunden sind laut NMR-Analyse im Rohgemisch bei 60prozentigem Umsatz neben Ausgangsketon die Verbindung Nr. 1 (R*, S*) und die hierzu diastereomere Verbindung (R*, R*) im Verhältnis 7:3 vorhanden.

c) 280 g 2,2-Dimethyl-4-(1,2,4-triazol-1-yl)-8-phenoxyoctanon-(3) werden in 1,8 l Dioxan gelöst und nach Zusatz von 10 g $RuO(OH)_x$ 62 Stunden im Autoklav unter 25 bar Wasserstoffdruck bei 125°C hydriert. Nach dem Abfiltrieren des Hydrierkatalysators und Abdestillieren des Lösungsmittel bleiben 274 g Rückstand, der laut NMR-Analyse neben 5% Ausgangsketon die Verbindung Nr. 1 (R*, S*) und die hierzu diastereomere Verbindung (R*, R*) im Verhältnis 7:3 enthält. Durch Kristallisation aus Diisopropylether kann hieraus die Verbindung Nr. 1 isoliert werden.

Beispiele für die Diastereomeren der Formel I mit R*, S*-Konfiguration, die in entsprechender Weise

hergestellt werden können, enthält die Tabelle 1. Die neuen R*,S*-Diastereomeren unterscheiden sich von den z.T. bekannten R*,R*-Diastereomeren hinsichtlich verschiedener chemischer und physikalischer Eigenschaften, z.B. hinsichtlich des Schmelzpunktes, der im allgemeinen für die neuen R*,S*-Diastereomeren höher liegt als für die jeweils entsprechenden R*,R*-Diastereomeren (siehe Tabelle 1). Generell sind die neuen R*,S*-Diastereomeren dadurch charakterisiert, daß sie sich bei der dünnschichtchromatographischen Analyse im System Kieselgel/Dichlormethan-Aceton 9:1 als deutlich polarer erweisen und einen geringeren $R_f$-Wert aufweisen als die jeweils entsprechenden R*,R*-Diastereomeren.

Weiterhin sind die in Deuterochloroform aufgenommenen $^1$H-NMR-Spektren der R*,S*-Diastereomeren durchweg dadurch charakterisiert, daß das Signal für die 9 Protonen der tert.-Butylgruppe bei 0,9—1.0 ppm auftritt, während das entsprechende Signal für die R*,R*-Diastereomeren bei 0,7—0,8 ppm liegt. Aus den entsprechenden integrierten Signalintensitäten können somit leicht die Anteile der Diastereomeren $^1$H-NMR-spektroskopisch ermittelt werden.

An den Beispielen der Verbindung Nr. 1 mit R*,S*-Konfiguration und des entsprechenden Diastereomeren mit R*,R*-Konfiguration wurden die relativen Konfigurationen der Diastereomeren mit Hilfe der Röntgenstrukturanalyse ermittelt.

Zusätzlich zu den neuen R*,S*-Diastereomeren enthält Tabelle 1 einige zu Vergleichszwecken durch Reduktion mit $NaBH_x$ hergestellte R*,R*-Diastereomere (Herstellung entsprechend Beispiel 2 aus EP—A—00 40 350).

Tabelle 1

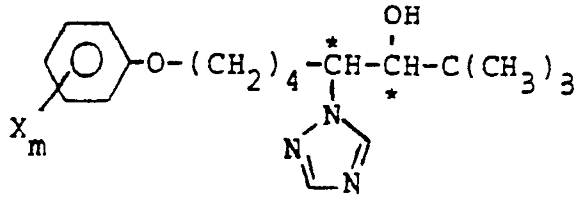

(I)

| Verb. Nr. | $X_m$ | R*,S*-Diastereomer % lt. NMR | Fp.(°C) | R*,R*-Diastereomer % lt. NMR | Fp.(°C) | EP-A-0040350 Beispiel |
|---|---|---|---|---|---|---|
| 1 | H | 90 % | 132-133 | 90 % | 79-81 | 2 |
| 2 | 2-Cl | > 95 % | 106 | > 95 % | 98 | - |
| 3 | 2-F | > 95 % | 89 | > 95 % | 87 | - |
| 4 | 3-Cl | > 95 % | 97 | > 95 % | 90 | - |
| 5 | 4-Cl | > 95 % | 159 | 90 % | 76-78 | 20 |
| 6 | 4-F | > 95 % | 126-128 | > 95 % | 86-88 | 77 |
| 7 | 4-$CH_3$ | > 95 % | 128-130 | > 90 % | Harz | - |
| 8 | 4-Methoxy | > 95 % | 108 | > 95 % | 89 | - |
| 9 | 3,5-$Cl_2$ | > 95 % | 122 | - | - | - |
| 10 | 3-$CF_3$ | > 95 % | 79-83 | - | - | - |
| 11 | 2-$CH_3$ | > 95 % | 89-93 | - | - | - |
| 12 | 2,6-$Cl_2$ | > 95 % | 113-116 | - | - | - |
| 13 | 2-Methoxy | > 95 % | 87-88 | - | - | - |
| 14 | 3-$CH_3$ | > 95 % | 75-79 | - | - | - |
| 15 | 3-Methoxy | > 95 % | 64-67 | - | - | - |
| 16 | 3-Phenyl | > 95 % | 110-111 | - | - | - |
| 17 | 4-Phenoxy | > 95 % | | - | - | - |
| 18 | 3-tert.-Butyl | > 95 % | 59-60 | - | - | - |
| 19 | 3-(1-Butoxy)- | > 95 % | 60-61 | - | - | - |
| 20 | 3-Isopropyl | > 95 % | 73-74 | - | - | - |
| 21 | 4-Isopropyl | > 95 % | 63-66 | - | - | - |
| 22 | 2,4-Dichlor | > 95 % | 106-107 | > 90 % | 91-93 | 39 |

Die vorteilhafte fungizide Wirksamkeit der Diastereomeren mit der Konfiguration R*,S* (im folgenden als B bezeichnet) gegenüber den Diastereomeren mit der Konfiguration R*,R* (im folgenden als A bezeichnet) wird aus den Vergleichsbeispielen ersichtlich.

Vergleichsbeispiel 1

Wirksamkeit gegen Alternaria solani an Tomaten

Im Gewächshaus kultivierte Topfpflanzen der Sorte "Große Fleischtomate" werden im Vierblattstadium mit wäßriger Suspension bespritzt, die aus der Trockensubstanz — bestehend aus 80% Wirkstoff und 20% Emulgator — aufbereitet wird. Nach dem Antrocknen des Spritzbelages werden die Blätter mit einer wäßrigen Sporensuspension des Pilzes Alternaria solani inoculiert. Diese Pflanzen werden dann in eine wasserdampfgesättigte Kammer mit Temperaturen zwischen 22° und 24°C gestellt. Nach 4 Tagen hat sich die Krankheit auf den unbehandelten, jedoch inoculierten Pflanzen so stark entwickelt, daß die fungizide Wirksamkeit der Substanzen beurteilt werden kann.

Bewertung:

0 = kein Pilzbefall, abgestuft bis

5 = Totalbefall

| Wirkstoff-Nr. (= Verbindung Nr.) | Befall der Blätter nach Applikation von 0,05 %iger Wirkstoffbrühe |
|---|---|
| 2 Diastereomeres B | 0 |
| - das korrespondierende Diastereomere A | 1 |
| 3 Diastereomeres B | 0 |
| - das korrespondierende Diastereomere A | 2 |
| 4 Diastereomeres B | 1 |
| - das korrespondierende Diastereomere A | 3 |
| 7 Diastereomeres B | 1 |
| - das korrespondierende Diastereomere A | 3 |
| 10 Diastereomeres B | 1 |
| - das korrespondierende Diastereomere A | 3 |
| 11 Diastereomeres B | 0 |
| - das korrespondierende Diastereomere A | 2 |
| 13 Diastereomeres B | 1 |
| - das korrespondierende Diastereomere A | 3-4 |
| unbehandelt | 5 |

Vergleichsbeispiel 2

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Bewertung:

0 = kein Pilzbefall, abgestuft bis

5 = Totalbefall

| Wirkstoff-Nr. (= Verbindung Nr.) | Befall der Blätter nach Applikation von ... %iger Wirkstoffbrühe | | | |
|---|---|---|---|---|
| | 0,05 | 0,025 | 0,0125 | 0,006 |
| 2 Diastereomeres B | 0 | 0 | 0 | 0 |
| - das korrespondierende Diastereomere A | 0 | 1 | 1 | 3 |
| 3 Diastereomeres B | 0 | 0 | 0 | 0 |
| - das korrespondierende Diastereomere A | 0 | 0 | 1 | 1 |
| 4 Diastereomeres B | 0 | 0 | 0 | 0 |
| - das korrespondierende Diastereomere A | 0 | 1 | 2 | 2 |
| 7 Diastereomeres B | 0 | 0 | 0 | 0 |
| - das korrespondierende Diastereomere A | 0 | 1 | 4 | 5 |
| 11 Diastereomeres B | 0 | 0 | 0 | 1 |
| - das korrespondierende Diastereomere A | 5 | 4 | 5 | 5 |
| 14 Diastereomeres B | 0 | 1 | 0 | 1 |
| - das korrespondierende Diastereomere A | 2 | 1 | 1 | 4 |
| 15 Diastereomeres B | 0 | 1 | 2 | 5 |
| - das korrespondierende Diastereomere A | 5 | 5 | 5 | 5 |
| 22 Diastereomeres B | 0 | 1 | 1 | 1 |
| - das korrespondierende Diastereomere A | 3 | 2 | 4 | 4 |
| unbehandelt | 5 | | | |

Vergleichsbeispiel 3

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Bewertung:

0 = kein Pilzbefall, abgestuft bis

5 = Totalbefall

| Wirkstoff-Nr. (= Verbindung Nr.) | Befall der Blätter nach Applikation von ... %iger Wirkstoffbrühe 0,025 | 0,003 |
|---|---|---|
| 10 Diastereomeres B | 0 | 0 |
| - das korrespondierende Diastereomere A | 0 | 1 |
| 11 Diastereomeres B | 0 | 2 |
| - das korrespondierende Diastereomere A | 2 | 4 |
| 13 Diastereomeres B | 1 | 3-4 |
| - das korrespondierende Diastereomere A | 4 | 4 |
| 14 Diastereomeres B | 0 | 0 |
| - das korrespondierende Diastereomere A | 1 | 3-4 |
| 15 Diastereomeres B | 0 | 3 |
| - das korrespondierende Diastereomere A | 4 | 4 |
| unbehandelt | 4-5 | |

Die erfindungsgemäßen Diastereomerengemische werden als fungizide Wirkstoffe, insbesondee im Pflanzenschutz, angewendet, indem man die Substrate bzw. Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Wirkstoffe dieser Erfindung können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die Wirkstoffe können auch im Materialschutz u.a. zur Bekämpfung holzzerstörender Pilze wie Coniophora puteana und Polystictus versicolor eingesetzt werden. Die Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 2 mit 10 Gewichtsteilen N-Methyl-alphapyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 7 werden mit 3 Gewichtsteilen des Natriumsalzes der Diisobutylnaphthalin-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 4 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 7 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die Wirkstoffe dieser Erfindung kombiniert werden können, soll die Kombinationsmöglichkeiten erläutern, nicht aber einschränken:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol

N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid

2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin

N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Die Wirkstoffe dieser Erfindung und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten, Basidiomyceten und Deuteromyceten, aus. Sie sind zum Teil systemisch wirksam und können als Blatt- und Bodenfungizide eingesetzt werden.

Besonders interessant sind die Wirkstoffe dieser Erfindung für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Garten- und Weinbau, sowie Gemüse — wie Gurken, Bohnen und Kürbisgewächse —.

Die Wirkstoffe dieser Erfindung sind insbesondere geeignet zur Bekämpfung folgender Pflanzenkrankheiten:

Erysiphe graminis (echter Mehltau) in Getreide,

Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten,
Verticillium-Spezies an Baumwolle und Gemüse.

Besonders hervorzuheben ist die Wirksamkeit gegen Botrytis und Alternaria.

**Patentansprüche**

1. Verfahren zur Herstellung von Mischungen, enthaltend Triazolylalkohole der Formel I

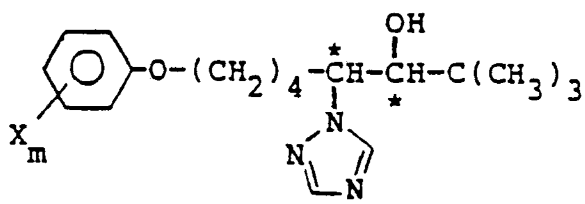

(I)

in der
X ein Wasserstoffatom, ein Halogenatom, Trifluormethyl, $C_1$- bis $C_4$-Alkyl, $C_1$- bis $C_4$-Alkoxy, gegebenenfalls substituiertes Phenyl oder gegebenenfalls substituiertes Phenoxy und
m eine ganze Zahl von 1 bis 5 bedeutet,
wobei X gleich oder verschieden sein kann, wenn m größer als 1 ist, die weniger als 30%, bezogen auf das Gesamtgemisch, an Diastereomeren, die an den beiden Chiralitätszentren die Konfiguration R*, R* haben, oder an Enantiomeren, die an den beiden Chiralitätszentren die Konfigurationen R,R oder S,S haben, enthalten, von deren Säureadditionssalzen oder deren Metallkomplexen dadurch gekennzeichnet, daß man Ketone der Formel II

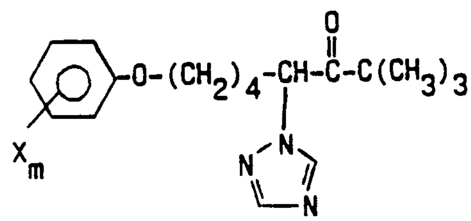

(II)

in der X und m die oben angegebene Bedeutung haben, mit sekundären Alkoholaten, mit Alkylmagnesiumhalogeniden, die einen Alkylrest mit 2 bis 6 C-Atomen und ein betaständiges Wasserstoffatom im Alkylrest enthalten, oder mit Wasserstoff in Gegenwart von Ruthenium oder Rutheniumderivaten und in Anwesenheit eines Verdünnungsmittels stereoselektiv reduziert und gegebenenfalls die so erhaltenen Mischungen in die Säureadditionssalze oder Metallkomplexe überführt.

2. Verfahren nach Anspruch 1, dadurch gekennzeichnet, daß die Hydrierung in Gegenwart von Rutheniumoxidhydrat vorgenommen wird.

3. Fungizides Mittel, enthaltend eine gemäß Anspruch 1 hergestellte Mischung und enthaltend zusätzlich einen festen oder flüssigen Trägerstoff.

4. Verfahren zur Bekämpfung von Schadpilzen, dadurch gekennzeichnet, daß man eine gemäß Anspruch 1 hergestellte Mischung auf die Pilze oder auf durch Pilzbefall bedrohte Substrate, Pflanzen oder Saatgüter einwirken läßt.

**Revendications**

1. Procédé pour la préparation de mélanges contenant des triazolylalcools de la formule I

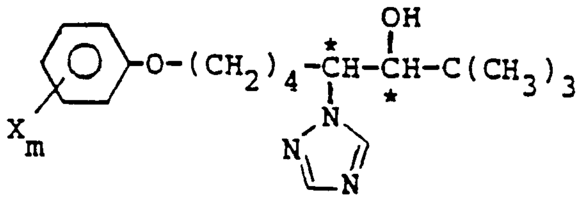

(I)

dans laquelle

X représente un atome d'hydrogène, un atome d'halogène, trifluorométhyle, alkyle en $C_1$ à $C_4$, alcoxy en $C_1$ à $C_4$, phényle éventuellement substitué ou phénoxy éventuellement substitué, et

m un nombre entier de 1 à 5

X pouvant être les mêmes ou différents, quand m est supérieur à 1, qui contiennent, de leurs sels d'addition d'acide ou de leurs complexes métalliques, moins de 30%, rapportés au mélange total, de diastéreoisomères, qui ont la configuration R*, R* à leurs deux centres de chiralité, ou d'énantiomères, qui ont les configurations R, R ou S, S à leurs deux centres de chiralité, caractérisé par le fait qu'on réduit stéréosélectivement des cétones de formule II

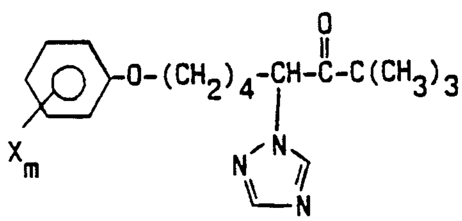

(II)

dans laquelle X et m ont les significations indiquées plus haut, avec des alcoolates secondaires, avec des halogénures d'alkylmagnésium qui contiennent un reste alkyle ayant 2 à 6 atomes C et un atome d'hydrogène en position bêta dans le reste alkyle, ou avec de l'hydrogène, en présence de ruthénium ou de dérivés de ruthénium, et en présence d'un diluant, et on transforme éventuellement les mélanges obtenus en les sels d'addition d'acide ou complexes métalliques.

2. Procédé selon la revendication 1, caractérisé par le fait que l'hydrogènation est effectuée en présence d'hydrate d'oxyde de ruthénium.

3. Agent fongicide, contenant un mélange préparé selon la revendication 1 et contenant additionnellement un support solide ou liquide.

4. Procédé de lutte contre des champignons nuisibles, caractérisé par le fait que l'on fait agir un mélange préparé selon la revendication 1 sur les champignons ou les substrats, plantes ou semences menacées d'une attaque par les champignons.

**Claims**

1. A process for the preparation of a mixture containing triazolyl alcohols of the formula I

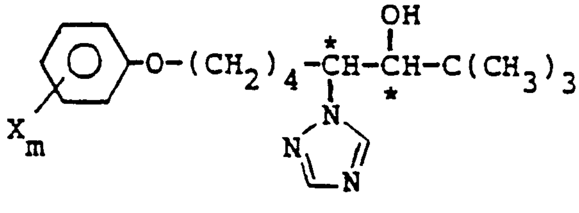

(I)

where

X is hydrogen, halogen, trifluoromethyl, $C_1$—$C_4$-alkyl, $C_1$—$C_4$-alkoxy, unsubstituted or substituted phenyl or unsubstituted or substituted phenoxy and

m is an integer of from 1 to 5,

and X may be identical or different when m is greater than 1,

which contains less than 30%, based on the total mixture, of diastereomers which have the configuration R*, R* at the two centers of chirality, or of enantiomers which have the configuration, R,R or S,S at the two centers of chirality, or acid addition salts or metal complexes thereof, wherein a ketone of the formula II

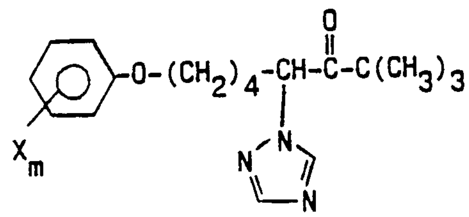

(II)

where X and m have the abovementioned meanings, is subjected to stereoselective reduction with a secondary alcoholate, with an alkylmagnesium halide which contains an alkyl of 2 to 6 carbon atoms and a beta-hydrogen atom in the alkyl radical, or with hydrogen in the presence of ruthenium or of a ruthenium derivative and in the presence of a diluent, and, if required, the mixture thus obtained is converted into the acid addition salts or metal complexes.

2. A process as claimed in claim 1, wherein the hydrogenation is carried out in the presence of hydrated ruthenium oxide.

3. A fungicide containing a mixture prepared as claimed in claim 1 and additionally containing a solid or liquid carrier.

4. A method for controlling harmful fungi, wherein a mixture prepared as claimed in claim 1 is allowed to act on the fungi or on substrates, plants or seed threatened by fungal attack.